# EUROPEAN PATENT SPECIFICATION

(11) **EP 4 570 161 B1**
(45) Date of publication and mention of the grant of the patent: **29.07.2026**
(21) Application number: 23217126.4
(22) Date of filing: 15.12.2023
(51) Int. Cl.: A61B 3/032, A61B 3/00, A61B 3/02, A61B 3/024

(54) **DEVICE FOR EYESIGHT EXAMINATION, IN PARTICULAR FOR PERFORMING FIELD OF VISION TESTS**
GERÄT ZUR AUGENUNTERSUCHUNG, INSBESONDERE ZUR DURCHFÜHRUNG VON SEHFELDTESTS
DISPOSITIF D'EXAMEN DE LA VUE, NOTAMMENT POUR LA RÉALISATION DE TESTS SUR LE TERRAIN DE LA VISION

(43) Date of publication of application: 18.06.2025
(73) Proprietor: OPTOPOL Technology Spolka z ograniczona Odpowiedzialnoscia, 42-400 Zawiercie (PL)
(72) Inventor: Bogdani, Szymon, 31-559 Kraków (PL); Stepien, Konrad, 43-100 Tychy (PL); Dziepak, Paulina, 51-180 Wroclaw (PL)
(74) Representative: AOMB Polska Sp. z.o.o.

(56) References cited:
- WO-A1-2023/285541
- CA-A1- 2 705 814
- KR-A- 20030 059 700
- US-A- 6 045 227
- US-A1- 2013 063 496
- US-A1- 2021 259 539

## Description

### Field of the Invention

The invention concerns a device designed for eyesight examination, which can be applied in medicine, in particular in ophthalmology. The device can be used for perimetry or visual field testing but also other parameters where tests are conducted by means of light stimuli displayed in front of the patient's eyes.

### Similar prior art

From the description included in US5864384A, we know a device that uses Virtual Reality for visual field testing. The device enables delivering, measuring and quantifying visual information to and from the visual pathways of the eye of a patient, their retina and the optic nerve. A screen being a part of the device covers a possibly large visual field of the patient. A computer connection allows for controlling the device and selecting the tests and stimuli delivered to the patient. Additionally, a switch or button is available for collecting responses from the patient. The use of a screen as a stimulus source allows displaying the fixation point at any location as well as changing the background, brightness, contrast, colours, size, details and duration of the optical stimuli. The use of such a device does not require us to adapt the entire room for testing, as the whole visual field of the patient is enclosed in the device. Another piece of the gear are goggles with independent eyepieces allowing the patient to observe a field of view into which test stimuli are presented to one eye at a time. The device uses Virtual Reality and is mounted on the patient's head.

From the description in WO2012148983A2, we know the use of two LCD panels in a screen for improving the contrast of the image produced. The use of two LCD panels together with a polarizer system allows achieving a high dynamic range (HDR). This way we increase the dynamic range of the device but at the same time reduce the image brightness, which is why a brightness increasing element is additionally used. The first screen in the presented technology is a colour screen that generates the image, while the other is an achromatic screen increasing the contrast.

From the description in WO2018169330A1, we know the use of a visual field measuring method. The method uses the Goldmann Ill stimulus with various brightness levels that is displayed to the patient at least once in different sections of the screen. The device is a head-mounted screen that generates and displays the stimulus. It also comprises a button that enables receiving the patient's responses, and the device itself collects and stores information about the responses. Then it transmits it to an electronic device that identifies a sequence of the user's responses and analyses and generates a report on the patient's visual field.

From the description in US2013/063496A1, we know the several embodiments of display systems that comprise a backlight source, a first modulator, a second modulator and a controller. A display system employing an edge-lit backlight with a field sequential illumination scheme using two or more sets of color primary emitters and dual monochrome LCD modulators, which allows for wider color gamut and reduced processing costs by eliminating the need for a high-resolution array of LEDs. The system achieves high dynamic range and wide color gamut with reduced processing requirements and costs, while minimizing color break-up and enhancing 3D visual effects through autostereoscopic and active shutter technologies.

From the description in US6045227A, we know the multi-functional, visual test instrument realized by integrating miniature, close-proximity displays, such as LCDs, with viewing optics, which is constructed in the form of preferably a unitary housing. The multi-functional visual test instrument featuring miniature, close-proximity displays integrated with viewing optics within a unitary housing, mounted on a movable base or arm, allowing for comfortable positioning without encircling the head, and incorporating computer-generated test stimuli and feedback mechanisms for efficient testing under ordinary light conditions. The solution provides a hygienic and comfortable visual testing experience by eliminating the need for shared head-worn instruments, allowing for effective testing of various visual functions like perimetry and acuity without disturbing the patient's hair or requiring head-worn devices, while maintaining the effectiveness of prior art instruments.

US 2013/063496 A1 discloses high dynamic range displays comprising MEMS/IMOD components. D2 describes a display system with components arranged in a specific order: illumination layer, brightness enhancing film, first polarization layer, first liquid crystal modulation layer, second polarization layer, second liquid crystal modulation layer, and third polarizing layer, wherein the first and second liquid crystal modulation layers comprise monochromatic LCD panels.

Unfortunately, the solutions existing in prior art show imperfections: the brightness levels achievable by the displays are often too low to perform an accurate eye examination. What is more, they have an unsatisfactory number of different brightness levels of the displayed points where we can conduct the tests, which also adversely affects the accuracy/quality of the tests. Finally, some of the available devices are inconveniently heavy and unnecessarily energy-consuming.

### Purpose of the invention

The purpose of the invention is therefore to provide a human eye examination device that would allow for performing diverse eye tests at a broad brightness range, that would be lightweight, small and not uncomfortable to operate, and that would show reduced energy consumption during operation.

### The essence of the invention

The invention as defined in independent claim 1 concerns an eye examination device that comprises:
- a housing with a shape suited to contain components of the device that are listed below and to accommodate the head, particularly the face, of the user for the period of test,
- powering means, for supplying the device with electrical energy,
- a display unit for generating light signals to perform tests,
- an optical system for transmitting the light signals from the display unit to the patient,
- a cooling system of the display unit,
characterised in that the display unit has the following components arranged in the order given below:
an illumination layer,
at least one brightness enhancing layer,
a first-type polarization layer,
a first liquid crystal modulation layer,
a second-type polarization layer,
a second liquid crystal modulation layer,
a first-type polarization layer,
wherein the first and second liquid crystal modulation layers are each provided with an 8-bit monochromatic LCD panel.

Preferred embodiments of the invention are defined in the appended claims 2-15.

Preferably, the illumination layer is selected from a group that includes: an LED sheet connected to light directing films, and a CCFL panel.

Preferably, the first-type and second-type polarization layers are characterised with a uniform transmission within the spectral range of visible light, i.e. with a transmission deviation lower than 10%.

Preferably, both LCD panels have an optical resolution configured and suitable for producing a light stimulus with an area that fits within a solid angle of 4.4*10⁻⁵ steradians +20%/-15%.

Preferably, the device comprises two display units as defined in claim 4, and also two optical systems and two cooling systems - one of each provided for each of the two display units, with one display unit, optical system and cooling system being provided for each eye.

Preferably, the optical system is provided with correction lenses arranged respectively for each eye, which are positioned in such a way to be axially movable relative to the tested eye, independently from each other.

Preferably, both display units are provided with means for moving each of them relative to the tested eye independently from each other.

Preferably, the optical system/s is/are provided with means for adjusting the position of the display unit/s and/or correction lenses, for moving it/them in horizontal direction in order to adjust it/them to the user's interpupillary distance.

Preferably, the cooling system/s is/are configured in such a way that over 50% of the coolant used for cooling the display unit is used for cooling both LCD panels, in particular over 2/3 of the coolant are used for cooling both LCD panels, whereas the remainder of the coolant is used for cooling the illumination layer.

Preferably, the housing is provided with an adjustable grip for mounting the device directly on the patient's head.

Preferably, the housing is additionally provided with connecting means for mounting the device to a stand.

Preferably, the powering means are provided both with an electrical connection for powering from the mains and with batteries or accumulators directly connected to the device.

Preferably, the housing has, at the spot where the patient brings their face closer, a replaceable and flexible gasket for blocking the light that comes from outside the device during examination.

Preferably, the display unit is suited and configured for achieving a brightness of 10000 asb +25%/-20%.

Preferably, the device has a digital computing system for controlling the device operation and for eye examination, which is directly integrated within the device.

The described device according to the invention meets the purpose defined above, considerably improving the capabilities and convenience of eye examination.

### Description of drawing figures

Fig. 1 shows an example solution for the device screen according to the invention,
Fig. 2 shows an example solution for an optical system projecting the image at the fundus of the patient's eye,
Fig. 3 shows the section of an example device that comprises the example optical system shown in Fig. 2,
Fig. 4 shows the device according to the invention positioned on a stand, in three views,
Fig. 5 shows the device according to the invention positioned on a stand, with a visible head grip, in three views.

### List of numerical symbols:

1 - screen of the device according to the invention / display unit
2 - illumination layer,
3 - brightness enhancing layer
4a, 4b - first-type and second-type polarization layers
5a, 5b - first and second modulation layers (e.g., LCD panel)
6 - optical system
7 - first lens from the optical system
8 - position adjustment direction of the first lens
9 - head of the device according to the invention
10 - stand for the device according to the invention
11 - chin rest
12 - adjustable head grip

### Preferred embodiments

The perimetry performed with the use of LCDs is known and used in miniaturized perimeters. A proper visual field test requires a high dynamic range and high brightness of the stimulus, which is difficult to achieve by existing devices.

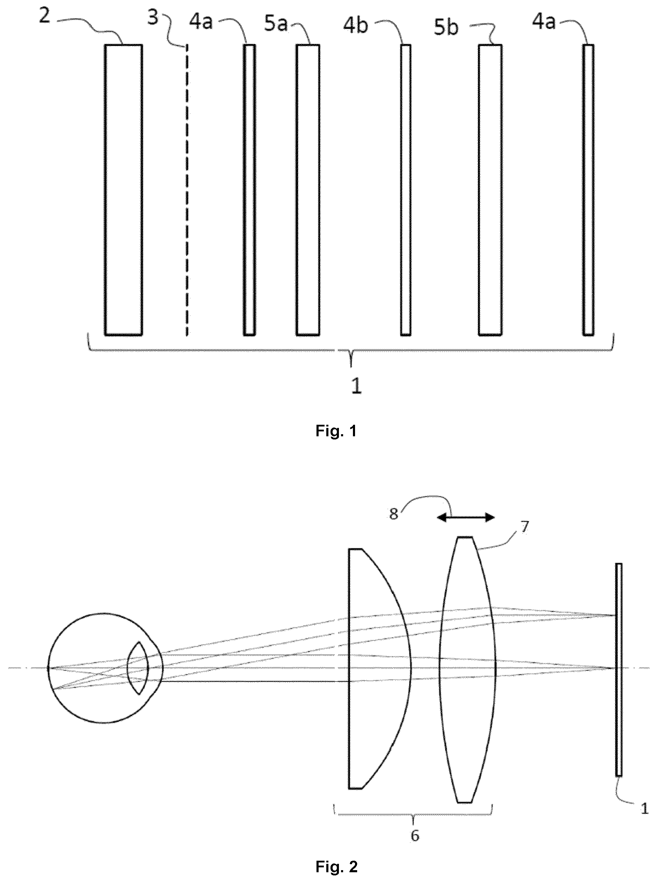

This issue is addressed by the screen 1 using two modulation layers 5a, 5b, in the form of 8-bit monochromatic LCD panels, that generate an image along with a set of polarization layers 4a, 4b, preferably in the form of polarizers, which in combination gives a good representation of black colour. The screen 1 uses an illumination layer 2, a brightness enhancing element 3, and a set of three polarization layers 4a, 4b, the first of which is positioned in front of the first modulation layer 5a, the second between the LCD modulation layers 5a, 5b, and the last behind the second modulation layer 5b, which is shown in Fig. 1.

Such a configuration of the screen 1 allows for achieving a dynamic range achieved in conventional perimeters, that is between 0 and 48 dB. The high dynamic range is possible to achieve by using an additional light modulation element in the form of an LCD panel 5b.

The use of two LCD panels for modulation also provides a high dynamic resolution which makes it possible to precisely represent the brightness levels required for eye examination. The applied 8-bit monochromatic panels enable achieving 2⁸ (256) levels of brightness modulation each. The set of two panels in the screen 1 enables achieving 2¹⁶ (65536) brightness levels in the entire dynamic range of 0 to 48 dB. Such a dynamic resolution allows us to fulfil the requirements of EN ISO 12866 standard relating to perimetric testing, specifically the background luminance tolerance (+25%/-20% of the stated value) and the differential luminance of stimulus stimuli (+25%/-20% of the stated value).

Moreover, owing to the use of monochromatic LCD panels, the device will consume less energy than colour panels. This in turn makes it easier to cool down the device components, particularly the components of the screen 1.

The object of the invention allows for eye examination that consists of: kinetic and static perimetry, Amsler test, visual acuity test at a distance and contrast sensitivity test, pupillometry, and Hirschberg test, owing to the use of a screen 1 and providing the device with the optical system 6 shown in Fig. 2.

The optical system 6 enables observing the image in infinity, which makes it possible to perform tests other than perimetry, as mentioned above. The neutral tension of eye lens and observation at a distance is ensured by dioptric adjustment, which is possible with a lens 7 and its movement (cf. 8 in Fig. 2) along the system's optical axis, and by adjusting the spacing of optical means (lenses or, even earlier, the displays themselves) to the patient's interpupillary distance. Since the patient's eyes are positioned so that the optical axes are parallel and the eyes do not accommodate, look at the object (the displayed stimulus) in infinity, the patient tires out less during examination, which in turn translates into their results.

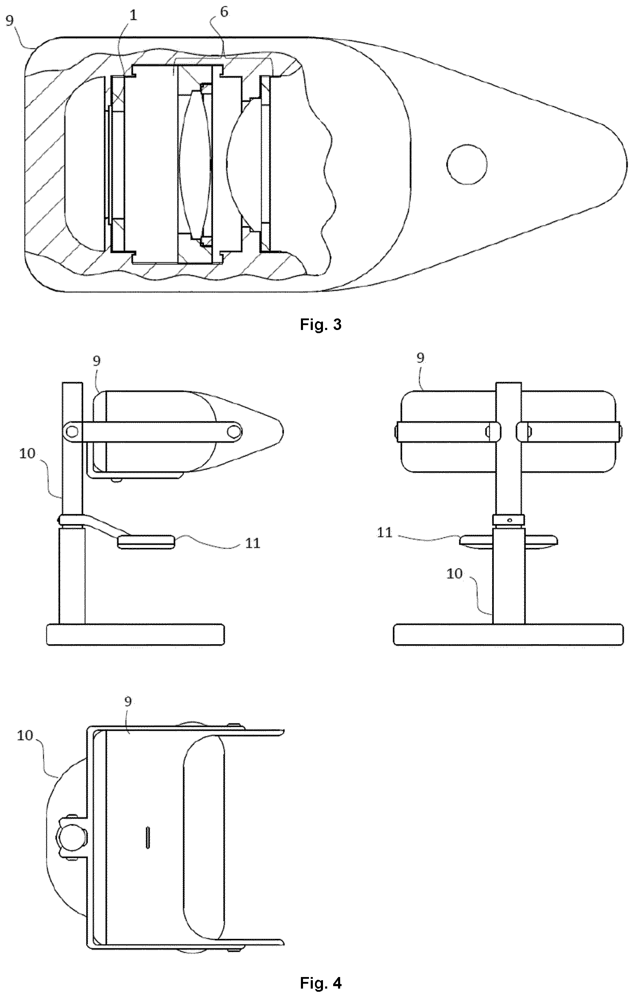

Fig. 3 shows sample positioning of the screen 1 and the optical system 6 inside the head 9 of the device according to the invention. Another factor that allows for conducting the tests, particularly perimetric tests, is the high contrast of the screen 1 due to the use of a screen 1 with two LCD panels 5a, 5b. The high contrast allows for maintaining correct results during visual acuity tests with optotypes and for proper recognition of structures, e.g. sinusoidal structures, while testing contrast sensitivity. With the use of an additional optical system 6, spherical correction of the refractive error is possible by dioptric adjustment at a range of, for example, -6 to 2 D. This allows us to carry out the perimetry properly, as for perimeters based on spherical and aspherical bowls, it is carried out at the appropriate correction of the patient (often by applying the correction lens in front of the tested eye). The said range of that adjustment covers majority of the population with refractive errors.

Performing a full kinetic perimetry requires the device to be capable of displaying the stimuli at the full Goldmann range, which presently corresponds to the generally accepted standards for the size and brightness of the stimuli. The object of the invention makes it possible with the use of a screen 1, a wide dynamic range of 0 to 48 dB, high dynamic resolution in the form of the achieved 65536 brightness levels, and a high maximum screen brightness of 10000 asb.

Owing to its size, the invention is a mobile device that can stand independently or be attached to the head. With that mobility, the device can be used both at doctor's offices and at the homes of patients with travel difficulties. The housing of the invention (including the possibility to use a flexible gasket installed to the housing at the place where the patient brings their face closer) and the method of installation cut off the patient's sight from ambient conditions, so the room needs not be additionally configured to properly perform the test (it can be performed in a room with the lights switched on / window curtains opened or, in an extreme case, even outdoors). The mobility also allows for examining patients in lying position if they are unable to sit in front of the device.

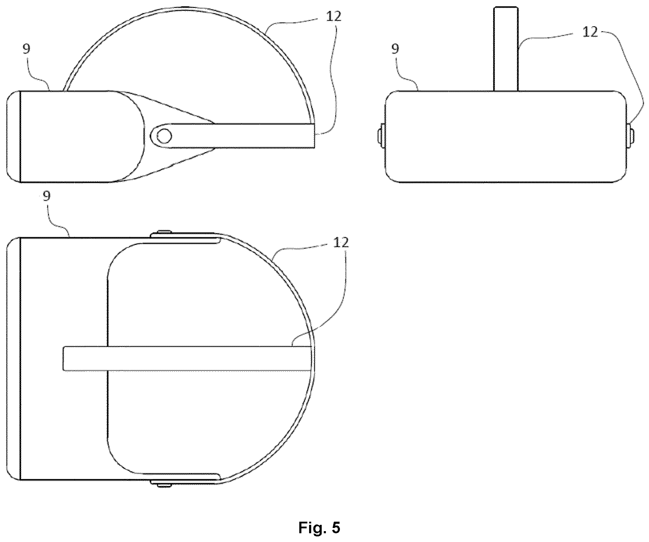

The object of the invention can be made as a device that enables operation in two modes: standing mode as in Fig. 4 and head-mounted mode as in Fig. 5. The standing mode, which can be used in doctor's offices, can be applied by positioning the head 9 of the device on a stand 10 that would additionally make it possible to adjust the height of the head 9 and of the chin rest 11. In this mode, the patient is steadily positioned against the screen 1 and optical system 6, and the device does not put the strain to the neck.

The modes can be switched by removing the head 9 from the stand 10 and by fitting the device with a head grip 12 (e.g., in the form of strips attached to the housing) that makes it possible to steadily fix the device on the head. In this mode, the patient can take any position without having to adjust the chin rest to the stand.

An embodiment of the screen 1 ensuring a high dynamic range is shown in Fig. 1. The illumination layer 2 illuminates two modulation layers 5a, 5b in the form of 8-bit monochromatic LCD panels generating an image that is displayed to the patient.

The LCD panels 5a, 5b are positioned between three polarization layers 4a, 4b that have the form of linear polarizers positioned relative to one another in such a way that polarizer 4b is in a cross arrangement with polarizers 4a. The polarizers 4a, 4b are positioned in the following sequence relative to one another: light-transmitting polarizer with type s polarization 4a, light-transmitting polarizer with type p polarization 4b, and light-transmitting polarizer with type s polarization 4a. The purpose of the former one is to linearly polarize any non-polarized light that comes from illumination 2 and is then transmitted to the first LCD 5a. The degree of light transmission through the LCD panel 5a depends on the orientation of the liquid crystal molecules in the display.

In one of the states of the panel, light polarization is modified by liquid crystal cells of the LCD 5a in such a way that light is transmitted through the polarizer 4b, being perpendicular to the first polarizer 4a, and illuminates the pixel at the second LCD panel 5b.

In the other state, light is blocked by the type p polarizer 4b, being perpendicular to the type s polarizer 4a, so it does not reach the corresponding pixel in the next LCD panel 5b.

Having passed through the type p polarizer 4b, the light falling onto the next LCD panel 5b illuminates, with an appropriate brightness, the pixels corresponding to the pixels of the first LCD panel 5a. Then the light with an adequately modified polarization passes through the next type s polarizer 4a being perpendicular to the type p polarizer 4b. At each of the polarizers 4a, 4b and LCD panels 5a, 5b, the light is increasingly more attenuated until achieving a high depth of black on the screen 1 at the spots where the pixels should be black.

Due to the losses in light intensity on elements of the screen 1, we used a brightness enhancing element 3 that is located between the illumination layer 2 and the first type s polarizer 4a. That element makes it possible to achieve an adequate brightness of the screen 1 for examination, even 10000 asb.

The image displayed through the screen 1 is projected on the patient's retina by the optical system 6 the embodiment of which is shown in Fig. 2. The purpose of that system is to form a beam of rays coming out of the screen 1 in such a way that its image is always produced at the fundus of the patient's eye. This is ensured by dioptric adjustment at the range of, for example, -6 to 2 D, which is implemented by movement 8 of the lens 7 along the optical axis of the system 6.

Below we present a hierarchical scheme of components of the device according to the invention, showing one by one the effects achieved by particular groups of features shown by the device.

In the simplest configuration, the device according to the invention, which is appropriate and adjusted for eye examination, requires the following components for operation: housing, powering means, display unit, optical system, and cooling system. In that basic version, it is very similar in terms of components to other devices known in prior art.

The modifications introduced as a result of long-term research and development distinguish the device according to the invention, specifically:
- the use of a display unit with the following structure according to the invention as defined in claim 1: illumination layer 2, brightness enhancing layer 3, first-type polarization layer (polarizer) 4a, first modulation layer (LCD panel) 5a, second-type polarization layer (polarizer) 4b, second modulation layer (LCD panel) 5b, first-type polarization layer (polarizer) 4a, ensures a brightness at the level required for examination and forms a structure with energy consumption and heat emission that does not damage the components (does not overheat the LCD panels) and can be cooled,
- the use of a system of two modulation layers (LCD panels) 5a, 5b according to the invention as defined in claim 1 that are monochromatic considerably restricts the possibility to use the discussed device and differentiates it from other, already known, displays positioned closely in front of the user's eyes (since VR sets, for example, are focused on the diversity of displayed colours), which, however, introduces a great benefit: it reduces energy consumption several times compared to similar devices having at least one colour LCD panel (and more than ten times compared to similar devices having both colour LCD panels, which were tested during the attempts to develop this solution); as a result, it turns out that instead of extensive cooling systems, which are heavy and tire out the user with the noise generated during operation, it is sufficient to use cooling systems that are smaller and user-friendly (lighter and more silent).

The illumination layer 2, having the form of a 8-bit monochromatic LED sheet, accompanied by light directing films, makes it possible to reduce the display unit in size (compared to the illumination with a single light source) and reduces energy consumption by the illumination layer 2, decreasing the amount of heat emitted on it and thus also the cooling demand.

Owing to said modifications, an optimisation of the cooling system is possible where two thirds of the coolant are allocated for cooling the LCD panels, or even where three fourths are allocated for cooling the LCD panels (and the remainder, for cooling the illumination layer 2).

The use of the first-type and second-type polarization layers (polarizers) 4a, 4b with a possibly uniform light transmission for the entire spectral range used (i.e., for visible light) is a beneficial modification that addresses the issue of the changing colour of displayed image depending on the brightness of the screen 1. The uniform transmission is defined as the lowest (that is, preferably, with a deviation lower than 10%) relation of transmission coefficient to light wave length. Said polarizers ensure a high brightness of the image while maintaining the illumination colour throughout the range of changes in brightness at which the screen 1 of the device according to the invention can operate.

Preferably, in some embodiments the use of one of the first-type polarizers 4a with a transmission of around 50% allows for increasing the brightness of the displayed image. The other polarizers should show a high extinction coefficient in order to achieve the minimum brightness required for examination.

The use of high-resolution LCD panels 5a, 5b provides a free selection of tests: a higher quantity of pixels improves the capability and accuracy of measurement (e.g., more possible areas/spots to illuminate while testing the visual field) and enables fulfilling the requirements of EN ISO 12866 relating to visual field testing, specifically the stimulus size tolerance (+20%/- 15% of the stated value converted into solid angle). It is difficult to unambiguously specify a single, precise, most beneficial resolution of the LCD panels 5a, 5b, as it will strictly depend on the device geometry, especially on the distance between the screen 1 and the user's eyes. Preferably, the value of the solid angle of the generated light stimulus should be 4.4*10⁻⁵ steradians. If we know the preferable guidelines expressed as the values of solid angle, we can adequately select the LCD panels for the already given, particular geometry of the device.

The display unit, optical system and cooling system, one for each eye, provides a great freedom and independence of measurements, where each eye can be examined in conditions adapted to it, without having to move the user away from the device and switch the device from measuring the left eye to measuring the right eye, etc.

The axially movable correction lenses provide easy correction of refractive errors in the patient's eye, so that the test can be performed without glasses, lenses at the cornea, etc.

The variant with the movable displays enables a similar correction of refractive errors in the patient's eyes without using lenses and with a reduced weight and size of the device.

Furthermore, the adjustable horizontal position of the displays/correction lenses is beneficial, as it allows for adjusting the space between these elements to the patient's interpupillary distance.

Providing the grip 12 for mounting the device on the patient's head significantly improves the freedom of using the device according to the invention, particularly by persons who find it difficult or inconvenient to sit down in front of the device placed on a table.

Providing the stand 10 and adjustment of the device housing to the two modes of measurements (with the device mounted on the head or on a table) considerably improves the mobility and freedom of operation.

Providing the two different powering means (from the mains and from batteries/accumulators) improve the device mobility and operating freedom even further.

Providing the flexible gasket in the housing, which is supposed to prevent the light from outside of the device against reaching the patient's eyes, increases the measuring comfort that will no longer be disturbed by the outside light or even by the light coming from the monitor used by the person conducting the test.

A device that can achieve a brightness of 10000 asb makes it possible to conduct tests according to a generally accepted standard for visual field testing devices.

A device that has its own computing system for controlling and performing eye examinations significantly improves the device independence and the possibility to use it as an autonomous and mobile device for eye examination.

One of other beneficial and commonly encountered modifications that can be included in the device according to the invention is also the fact that it is provided with means for monitoring the movements of eye pupil, for example in the form of a camera recording the motions of the eyeball during examination. Such an additional camera is beneficial, as it enables an ongoing view of the eye during test, which can provide the testing person with additional information about the condition of the patient's eyes.

The device may be configured so to display light stimuli in various configurations: a single stimulus or multiple stimuli at a time, according to the requirements, circumstances and nature of the test.

It should be indicated that the modifications discussed above can be used in various configurations.

## Claims

1. An eyesight examination device that comprises:
- a housing with a shape suited to contain components of the device that are listed below, and to accommodate the head, particularly the face, of the user for the period of test,
- powering means, for supplying the device with electrical energy,
- a display unit (1) for generating light signals to perform tests,
- an optical system (6) for transmitting the light signals from the display unit to the patient,
- a cooling system of the display unit,
**characterised in that** the display unit (1) has the following components arranged in the order given below:
an illumination layer (2),
at least one brightness enhancing layer (3),
a first-type polarization layer (4a),
a first liquid crystal modulation layer (5a),
a second-type polarization layer (4b),
a second liquid crystal modulation layer (5b),
a first-type polarization layer (4a),
wherein the first and second liquid crystal modulation layers (5a, 5b) are each provided with an 8-bit monochromatic LCD panel.

2. The device according to claim 1 **characterised in that** the illumination layer (2) is selected from a group that includes: an LED sheet connected to light directing films, and a CCFL panel.

3. The device according to claim 2, wherein the first-type and second-type polarization layers (4a, 4b, 4a) are characterised with a uniform transmission within the spectral range of visible light, i.e. with a transmission deviation lower than 10%.

4. The device according to claim 3, wherein both LCD panels (5a, 5b) have an optical resolution configured and suitable for producing a light stimulus with an area that fits within a solid angle of 4.4*10⁻⁵ steradians +20%/-15%.

5. The device according to claim 4, which comprises two display units (1) as defined in claim 4 and also two optical systems (6) and two cooling systems - one of each provided for each of the two display units (1), with one display unit (1), optical system (6) and cooling system being provided for each eye.

6. The device according to claim 4 or 5, wherein the optical system (6) is provided with correction lenses (7) arranged respectively for each eye, which are positioned in such a way to be axially movable relative to the tested eye, independently from each other.

7. The device according to claim 5, wherein both display units (1) are provided with means for moving each of them relative to the tested eye independently from each other.

8. The device according to claim 6 or 7, wherein the optical system/s (6) is/are provided with means for adjusting the position of the display unit/s (1) and/or correction lenses (7), for moving it/them in a horizontal direction in order to adjust it/them to the user's interpupillary distance.

9. The device according to claim 8, wherein the cooling system/s is/are configured in such a way that over 50% of the coolant used for cooling the display unit is used for cooling both LCD panels (5a, 5b), in particular over 2/3 of the coolant is used for cooling both LCD panels (5a, 5b), whereas the remainder of the coolant is used for cooling the illumination layer (2).

10. The device according to claim 9, wherein the housing is provided with an adjustable grip (12) for mounting the device directly on the patient's head.

11. The device according to claim 10, wherein the housing is additionally provided with connecting means for mounting the device to a stand (10).

12. The device according to claim 11, wherein the powering means are provided both with an electrical connection for powering from the mains and with batteries or accumulators directly connected to the device.

13. The device according to claim 11 or 12, wherein the housing has, at the spot where the patient brings their face closer, a replaceable and flexible gasket for blocking light that comes from outside the device during examination.

14. The device according to claim 13, wherein the display unit (1) is suited and configured for achieving a brightness of 10000 asb +25%/-20%.

15. The device according to claim 14, which has a digital computing system for controlling the device operation and for eye examination, which is directly integrated within the device.

## Patentansprüche

1. Gerät zur Untersuchung des Sehvermögens, welches Folgendes umfasst:
- ein Gehäuse mit einer Form, die geeignet ist, die nachstehend aufgeführten Komponenten des Geräts aufzunehmen und den Kopf, insbesondere das Gesicht, des Benutzers während der Testdauer aufzunehmen,
- eine Stromversorgungseinrichtung zur Versorgung des Geräts mit elektrischer Energie,
- eine Anzeigeeinheit (1) zur Erzeugung von Lichtsignalen zur Durchführung von Tests,
- ein optisches System (6) zur Übertragung der Lichtsignale von der Anzeigeeinheit zum Patienten,
- ein Kühlsystem für die Anzeigeeinheit,
**dadurch gekennzeichnet, dass** die Anzeigeeinheit (1) die folgenden Komponenten aufweist, die in der unten angegebenen Reihenfolge angeordnet sind:
eine Beleuchtungsschicht (2),
mindestens eine Helligkeitsverstärkungsschicht (3),
eine Polarisationsschicht des ersten Typs (4a),
eine erste Flüssigkristall-Modulationsschicht (5a),
eine Polarisationsschicht des zweiten Typs (4b),
eine zweite Flüssigkristall-Modulationsschicht (5b),
eine Polarisationsschicht des ersten Typs (4a),
wobei die erste und die zweite Flüssigkristall-Modulationsschicht (5a, 5b) jeweils mit einem 8-Bit-Monochrom-LCD-Panel versehen sind.

2. Gerät nach Anspruch 1, **dadurch gekennzeichnet, dass** die Beleuchtungsschicht (2) aus einer Gruppe ausgewählt ist, die Folgendes umfasst: ein mit Lichtleitfolien verbundenes LED-Sheet und ein CCFL-Panel.

3. Gerät nach Anspruch 2, wobei die Polarisationsschichten des ersten und zweiten Typs (4a, 4b, 4a) durch eine gleichmäßige Transmission innerhalb des Spektralbereichs des sichtbaren Lichts gekennzeichnet sind, d. h. mit einer Transmissionsabweichung von weniger als 10 %.

4. Gerät nach Anspruch 3, wobei beide LCD-Panels (5a, 5b) eine optische Auflösung aufweisen, die so konfiguriert und geeignet ist, einen Lichtreiz mit einer Fläche zu erzeugen, die in einen Raumwinkel von 4,4 × 10⁻⁵ Steradiant ±20 %/-15 % passt.

5. Gerät nach Anspruch 4, welches zwei Anzeigeeinheiten (1) gemäß Anspruch 4 sowie zwei optische Systeme (6) und zwei Kühlsysteme umfasst - jeweils eines für jede der beiden Anzeigeeinheiten (1), wobei für jedes Auge eine Anzeigeeinheit (1), ein optisches System (6) und ein Kühlsystem vorgesehen sind.

6. Gerät nach Anspruch 4 oder 5, wobei das optische System (6) mit jeweils für jedes Auge angeordneten Korrekturlinsen (7) versehen ist, die so positioniert sind, dass sie unabhängig voneinander axial relativ zum getesteten Auge bewegbar sind.

7. Gerät nach Anspruch 5, wobei beide Anzeigeeinheiten (1) mit Mitteln versehen sind, um jede von ihnen unabhängig voneinander relativ zum getesteten Auge zu bewegen.

8. Gerät nach Anspruch 6 oder 7, wobei das/die optische(n) System(e) (6) mit Mitteln zum Einstellen der Position der Anzeigeeinheit(en) (1) und/oder der Korrekturlinsen (7) versehen ist/sind, um diese in horizontaler Richtung zu bewegen und sie an den Pupillenabstand des Benutzers anzupassen.

9. Gerät nach Anspruch 8, wobei das/die Kühlsystem(e) so konfiguriert ist/sind, dass über 50 % des zur Kühlung der Anzeigeeinheit verwendeten Kühlmittels zur Kühlung beider LCD-Panels (5a, 5b) verwendet wird, insbesondere über 2/3 des Kühlmittels zur Kühlung beider LCD-Panels (5a, 5b) verwendet wird, während der Rest des Kühlmittels zur Kühlung der Beleuchtungsschicht (2) verwendet wird.

10. Gerät nach Anspruch 9, wobei das Gehäuse mit einem verstellbaren Griff (12) versehen ist, um das Gerät direkt am Kopf des Patienten zu befestigen.

11. Gerät nach Anspruch 10, wobei das Gehäuse zusätzlich mit Verbindungsmitteln versehen ist, um das Gerät an einem Ständer (10) zu befestigen.

12. Gerät nach Anspruch 11, wobei die Stromversorgungseinrichtungen sowohl mit einem elektrischen Anschluss zur Stromversorgung aus dem Netz als auch mit direkt an das Gerät angeschlossenen Batterien oder Akkus versehen sind.

13. Gerät nach Anspruch 11 oder 12, wobei das Gehäuse an der Stelle, an der der Patient sein Gesicht näherbringt, eine austauschbare und flexible Dichtung aufweist, um Licht abzublocken, das während der Untersuchung von außerhalb des Geräts einfällt.

14. Gerät nach Anspruch 13, wobei die Anzeigeeinheit (1) dafür ausgelegt und konfiguriert ist, eine Helligkeit von 10000 asb +25 %/-20 % zu erreichen.

15. Gerät nach Anspruch 14, welches ein digitales Rechensystem zur Steuerung des Gerätbetriebs und zur Augenuntersuchung aufweist, das direkt in das Gerät integriert ist.

## Revendications

1. Un dispositif d'examen de la vue qui comprend :
- un boîtier dont la forme est adaptée pour contenir les composants du dispositif énumérés ci-dessous et pour accueillir la tête, en particulier le visage, de l'utilisateur pour la durée du test,
- moyens d'alimentation, pour alimenter le dispositif en énergie électrique,
- une unité d'affichage (1) pour générer des signaux lumineux pour effectuer des tests,
- un système optique (6) pour transmettre les signaux lumineux de l'unité d'affichage au patient,
- un système de refroidissement de l'unité d'affichage,
**caractérisé en ce que** l'unité d'affichage (1) comprend les composants suivants, disposés dans l'ordre indiqué ci-dessous :
une couche d'éclairage (2),
au moins une couche améliorant la luminosité (3),
une couche de polarisation de premier type (4a),
une première couche de modulation à cristaux liquides (5a),
une couche de polarisation de deuxième type (4b),
une deuxième couche de modulation à cristaux liquides (5b),
une couche de polarisation de premier type (4a),
dans laquelle les première et deuxième couches de modulation à cristaux liquides (5a, 5b) sont chacune équipées d'un panneau LCD monochrome de 8 bits.

2. Le dispositif selon la revendication 1, **caractérisé en ce que** la couche d'éclairage (2) est choisie parmi un groupe comprenant : une feuille LED reliée à des films de guidage de la lumière, et un panneau CCFL.

3. Le dispositif selon la revendication 2, dans lequel les couches de polarisation de premier type et de deuxième type (4a, 4b, 4a) sont **caractérisées par** une transmission uniforme dans le domaine spectral de la lumière visible, c'est-à-dire par un écart de transmission inférieur à 10 %.

4. Le dispositif selon la revendication 3, dans lequel les deux panneaux LCD (5a, 5b) ont une résolution optique configurée et adaptée pour produire un stimulus lumineux dont la surface s'inscrit dans un angle solide de 4,4 × 10⁻⁵ stéradians + 20 % / - 15 %.

5. Le dispositif selon la revendication 4, qui comprend deux unités d'affichage (1) telles que définies dans la revendication 4, ainsi que deux systèmes optiques (6) et deux systèmes de refroidissement - un de chaque étant prévu pour chacune des deux unités d'affichage (1), une unité d'affichage (1), un système optique (6) et un système de refroidissement étant prévus pour chaque œil.

6. Le dispositif selon la revendication 4 ou 5, dans lequel le système optique (6) est pourvu de lentilles de correction (7) disposées respectivement pour chaque œil, lesquelles sont positionnées de manière à pouvoir se déplacer axialement par rapport à l'œil testé, indépendamment l'une de l'autre.

7. Le dispositif selon la revendication 5, dans lequel les deux unités d'affichage (1) sont pourvues de moyens permettant de les déplacer chacune par rapport à l'œil testé indépendamment l'une de l'autre.

8. Le dispositif selon la revendication 6 ou 7, dans lequel le ou les systèmes optiques (6) sont pourvus de moyens permettant de régler la position de la ou des unités d'affichage (1) et/ou des lentilles de correction (7), de les déplacer horizontalement afin de les adapter à la distance interpupillaire de l'utilisateur.

9. Le dispositif selon la revendication 8, dans lequel le ou les systèmes de refroidissement sont configurés de telle sorte que plus de 50 % du fluide de refroidissement utilisé pour refroidir l'unité d'affichage est utilisé pour refroidir les deux panneaux LCD (5a, 5b), en particulier plus de 2/3 du fluide de refroidissement est utilisé pour refroidir les deux panneaux LCD (5a, 5b), tandis que le reste du fluide de refroidissement est utilisé pour refroidir la couche d'éclairage (2).

10. Le dispositif selon la revendication 9, dans lequel le boîtier est pourvu d'un dispositif de maintien réglable (12) pour monter le dispositif directement sur la tête du patient.

11. Le dispositif selon la revendication 10, dans lequel le boîtier est en outre pourvu de moyens de fixation pour monter le dispositif sur un support (10).

12. Le dispositif selon la revendication 11, dans lequel les moyens d'alimentation sont pourvus à la fois d'une connexion électrique pour l'alimentation à partir du secteur et de batteries ou d'accumulateurs directement connectés au dispositif.

13. Le dispositif selon la revendication 11 ou 12, dans lequel le boîtier comporte, à l'endroit où le patient approche son visage, un joint d'étanchéité remplaçable et souple pour bloquer la lumière provenant de l'extérieur du dispositif pendant l'examen.

14. Le dispositif selon la revendication 13, dans lequel l'unité d'affichage (1) est adaptée et configurée pour atteindre une luminosité de 10 000 asb + 25 %/-20 %.

15. Le dispositif selon la revendication 14, qui comporte un système informatique numérique pour contrôler le fonctionnement du dispositif et pour l'examen des yeux, qui est directement intégré au dispositif.
